Europäisches Patentamt

European Patent Office

Office européen des brevets

(19)

(11) Veröffentlichungsnummer : **0 086 512**
**B1**

(12)

# EUROPÄISCHE PATENTSCHRIFT

(45) Veröffentlichungstag der Patentschrift :
15.04.87

(21) Anmeldenummer : 83200101.0

(22) Anmeldetag : 24.01.83

(51) Int. Cl.⁴ : **A 61 M 25/00**, A 61 J 1/00

(54) **Mehrschichtige, medizinische Arbeitsmittel.**

(30) Priorität : 11.02.82 DE 3204762

(43) Veröffentlichungstag der Anmeldung :
24.08.83 Patentblatt 83/34

(45) Bekanntmachung des Hinweises auf die Patenterteilung : 15.04.87 Patentblatt 87/16

(84) Benannte Vertragsstaaten :
AT BE CH FR GB IT LI LU NL SE

(56) Entgegenhaltungen :
DE-A- 1 491 682
DE-A- 1 930 136
DE-A- 2 622 502
GB-A- 2 009 359
US-A- 3 618 614
US-A- 4 119 267

(73) Patentinhaber : REHAU AG + Co
Rheniumhaus
D-8673 Rehau (DE)

(72) Erfinder : Kühlein, Georg
Wunsiedler Strasse 22
D-8671 Röslau (DE)
Erfinder : Gerlach, Ernst, Dr.
Dietrich-Bonhöfer-Strasse 1
D-8673 Rehau (DE)

**0 086 512**

**Beschreibung**

Die Erfindung betrifft technisches Zubehör für medizinische Arbeitsgeräte wie Schläuche, Behälter, Formteile mit mehrschichtigen Wandungen aus glasklarem, weichem Kunststoff, wobei wenigstens eine der Wandungen physiologisch einwandfrei und die anderen mit physiologisch bedenklichen und/oder ungeprüften Zuschlagstoffen, z. B. radioopaken Material, Farbbestandteilen, versehen sind.

Im medizinischen Arbeitsbereich richtet man sich bei der Zulässigkeit der Verarbeitung von Kunststoffen zu medizinischen Arbeitsgeräten nach der DIN 58 361 mit dem Titel « Transfusionsbehältnisse und Zubehör ». Im europäischen Bereich entspricht dieser DIN der Entwurf PA/PH/Exp. 3/T (80) 3 der European Pharmacopoeia Commission. Danach sind technische Arbeitsgeräte aus Kunststoff im medizinischen Bereich u. a. nur dann zulässig, wenn sie toxikologisch einwandfrei sind und weder Antioxydantien noch Farbmittel enthalten.

In diesem Zusammenhang ist die Verwendung von weichgemachtem Polyvinylchlorid mit dem Weichmacher Di-(2-Äthylhexyl)-Phthalat (DOP) zulässig.

Um die hohen Anforderungen bei der Verwendung von Kunststoffen zur Herstellung von medizinischen Arbeitsgeräten zu erfüllen, sind Verfahren bekannt geworden, welche die toxikologische Wirkung eines bestimmten, für die vorgesehene Verwendungsart besonders geeigneten Kunststoffes durch Beschichtung mit anderen, toxikologisch einwandfreien Kunststoffen verhindern sollen. So ist z. B. aus der DE-PS 19 30 136 ein beschichteter Katheter bekannt, welcher als Basis aus einem Schlauch aus Gummi oder einem anderen Elastomeren besteht und mit einem Überzug ihres hydrophylen Acrylats bzw. Methacrylats versehen ist.

Die Acrylat- oder Methacrylatschicht wird nach dem Anquellen des Katheters zu einem festen Überzug polymerisiert. Auf diese Schicht können antibiotische oder germizide Stoffe aufgebracht werden, welche von dem Hydrophilen Überzug festgehalten werden. Der Katheter wird damit *in situ* patientenverträglicher ausgestattet.

Ein ähnlicher Katheter ist aus der DE-PS 14 91 682 bekannt, bei dem die Außen- und die Innenoberflächen mit einer dünnen Schicht aus einer zu Elastomeren härtbaren Organopolysiloxan-Formmasse überzogen wurde und die Formmasse anschließend zu einem elastomeren Überzug gehärtet wird.

Schließlich ist aus der US-PS 36 18 614 ein medizinischer Schlauch bekannt, der aus einem relativ dicken, transparenten Innenschlauch und einem relativ dünnen, transparenten Außenschlauch — jeweils aus Kunststoff — besteht. Der Außenschlauch ist mit einem radioopaken Material versehen, so daß der Weg des Schlauches innerhalb des Patienten am Röntgenschirm verfolgt werden kann. Die geschützte Schlauchkombination besteht, z. B. aus einem Innenschlauch aus Polyäthylen mit Zuschlagstoffen wie Bismut-Oxychlorid- und Äthyl-2-(3-Amino-2,4,6-Triodobenzoyloxi)-Butanoat und einem Außenschlauch aus Polyäthylen mit Zuschlägen von Tri- und Tetraiodo-Benzosäure-Ester, welche als radioopakes Material homogen in der Wandung des Außenschlauches verteilt sind.

Dieser Schlauch ist unter Beachtung der DIN 58 361 nicht als medizinischer Schlauch verwendbar, da hier unzulässige Zuschlagstoffe zu den Basispolymeren verwendet werden.

Die Nachteile aus dem bekannten Stand der Technik bei der Herstellung toxikologisch einwandfreier Arbeitsgeräte für den medizinischen Anwendungsbereich bestehen darin, daß hier Herstellungsverfahren verwendet werden, welche technisch aufwendig und arbeitsintensiv sind.

Nach der Extrusion des Basisschlauches werden z. B. die toxikologisch einwandfreien Schichten durch einen weiteren Arbeitsgang wie Tauchen, Sprühen oder anderweitiges Beschichten aufgebracht. Bei der Verwendung von weichmacherhaltigen PVC-Materialien zur Herstellung der Schläuche wurden ferner die Wanderungsbestrebungen des Weichmachers bisher nicht beachtet, welche bis zur Versprödung der Schlauchwandungen führen können.

Kaum bekannt wurden bisher im medizinischen Arbeitsbereich technische Arbeitsgeräte, z. B. Schlauchsysteme, für lichtempfindliche Lösungen. Interessierte Verbraucher haben sich hier, entgegen den Vorschriften der DIN 58 361, recht und schlecht damit beholfen, daß schwarz oder dunkel eingefärbte Schlauchsysteme mit Zubehörteilen verwendet wurden, die zwar die Durchfluß substanz vor dem gesamten Wellenlängenbereich des sichtbaren wie des unsichtbaren Lichtes schützen, jedoch neben dem nicht DIN-gerechten Einsatz den Nachteil haben, daß das Durchflußgut nicht beobachtet werden kann. Auftretende Blasenbildung, Schmutzpartikel oder sonstige Störungen, die beispielsweise bei Infusionsvorgängen lebenswichtig für den Patienten sein können, sind mit solchen Arbeitsgeräten nicht direkt zu beobachten und können daher bei evtl. Auftreten nicht sofort abgestellt werden. Hier setzt die Erfindung ein, die sich zur Aufgabe gestellt hat, ein technisches, medizinisches Arbeitsgerät wie beispielsweise einen Schlauch aus Kunststoff zu schaffen, durch den ein lichtempfindliches Durchflußgut geleitet werden kann, ohne daß das Durchflußgut entweder von den Lichtwellen oder von den toxikologisch schädlichen Zuschlagstoffen geschädigt wird und bei dem darüber hinaus eine Transparenz erhalten bleibt, welche die Beobachtung des Durchflußgutes in jeder Phase des Durchflusses gewährleistet.

Wesentlich für die Aufgabenstellung ist noch, daß diese positiven Eigenschaften über eine längere Zeit erhalten bleiben müssen und nicht beispielsweise durch Weichmacherwanderung aufgehoben bzw.

gestört werden dürfen.

Diese Aufgabe wird bei technischem Zubehör für medizinische Arbeitsgeräte dadurch daß die physiologisch einwandfreie Wand jeweils einen höheren Weichmachergehalt besitzt als die dieser Wand benachbarte Wand, in der die physiologisch bedenklichen und/oder ungeprüften Zuschlagstoffe eingebracht sind, derart, daß das verstärkte Migrationsbestreben des Weichmachers aus der physiologisch einwandfreien Wand die Migration der physiologisch bedenklichen und/oder ungeprüften Zuschlagstoffe in die physiologisch einwandfreie Wand verhindert.

Bei der Verwendung weichmacherhaltigen Polyvinylchlorids hat sich als vorteilhaft herausgestellt, daß das Weichmachergefälle zwischen den Wandbereichen der physiologisch einwandfreien zur physiologisch bedenklichen Wandung im Verhältnis 1,1 : 1 bis 9 : 1 steht. Wird bei einem medizinischen Arbeitsmittel gemäß der Erfindung nur eine Wandung aus weich eingestelltem Polyvinylchlorid verwendet, während die andere oder die anderen Wandungen aus anderen weichen Polymeren gebildet sind, hat es sich als Zweckmässig erwiesen, daß der Weichmacheranteil der Wandung aus Polyvinylchlorid weich wenigstens 7 Gewichtsprozent beträgt.

Bei der Verwendung von Wandungen aus weich eingestelltem Polyvinylchlorid betragen die Gewichtsprozente bei einem Verhältnis von 1,1 : 1 annähernd 8 : 7, während die obere Grenze bei einem Verhältnis von 9 : 1 in Gewichtsprozent ausgedrückt bei 60 : 7 liegt. Die unterste Grenze wird dabei von einem Anteil von 7 Gewichtsprozent gebildet, ab dem grundsätzlich erst von Polyvinylchlorid weich gesprochen werden kann. Die oberste Grenze liegt bei 60 Gewichtsprozent, da ab diesem Wert das Polychlorid beginnt, klebrig zu werden.

Werden Wandungen aus anderen Polymeren zu einer Wandung aus weich gemachtem Polyvinylchlorid zugeordnet, muß ebenso der Mindest-Gewichtsprozent-Anteil des Weichmachers im Polyvinylchlorid 7 sein und der maximale Anteil 60.

Die physiologisch bedenklichen und/oder ungeprüften Zuschlagstoffe können strahlenabsorbierende Substanzen sein, wobei die Strahlen im Wellenlängenbereich des sichtbaren Lichtes genauso absorbiert werden wie die Strahlen im Wellenlängenbereich des unsichtbaren Lichtes. Durch spezielle Auswahl von Absorbersubstanzen können auch einzelne Strahlenbereiche des sichtbaren Lichtes gesondert herausgefiltert werden, sofern dies das Durchflußgut erforderlich macht. Es können auch solche Absorbersubstanzen als Zuschlagstoffe verwendet werden, welche Röntgenstrahlung absorbieren.

Solche Zuschlagstoffe können aber auch Vernetzungsmittel wie Peroxide oder Vernetzungshilfsmittel wie Strahlungsaktivatoren sein, wobei nach vollzogener Vernetzung physiologisch nicht einwandfreie Rückstände in der Schlauchwand verbleiben. Die Vernetzung kann dabei durch Energieaufnahme mittels Strahlung oder auf chemischem Wege erfolgen. Bei der Strahlenvernetzung kann zwar durch Zusatz von Vernetzungsaktivatoren die hohe Dosierung der normalen Strahlenvernetzung reduziert und damit ein teilweiser Materialabbau durch Strahlung, der zur Bildung toxischer Folgeprodukte führen kann, vermindert werden. Eine verringerte Bildung solcher toxischer Folgeprodukte ist jedoch auch bei einem Zusatz von Vernetzungsaktivatoren nicht auszuschließen. Bei der chemischen Vernetzung bleiben auf jeden Fall physiologisch bedenkliche Rückstände in der Wandung des vernetzten Kunststoffteils zurück. Diese Rückstände können durch das erfindungsgemäße Weichmachergefälle *in situ* neutralisiert werden. Durch die Vernetzung kann eine Verbesserung der Knickbeständigkeit, eine Optimierung des Rückstellvermögens und eine Verminderung des Kaltflusses bei konfektionierten Teilen wie Infusions-/Transfusionsbestecken erzielt werden.

Wird ein Schlauchaufbau aus weich eingestelltem Polyvinylchlorid verwendet, so kann dieser mehrschichtige Schlauchaufbau mit dem Weichmachergefälle zwischen zwei benachbarten Wandbereichen beispielsweise zum beobachtbaren Transport lichtempfindlicher Durchflußgüter verwendet werden. Durch das Weichmachergefälle zwischen den beiden benachbarten Schlauchwandungen entsteht bei der stärkeren Beaufschlagung des einen Wandbereichs mit Weichmacherbestandteilen gegenüber der schwächeren Beaufschlagung des anderen Wandbereichs mit Weichmacherbestandteilen ein osmoseähnlicher Zustand. Die vermehrten Weichmacherbestandteile des einen Wandbereiches bewegen sich dabei im Wege einseitiger Diffusion langsam in den benachbarten Wandbereich mit dem geringeren Weichmacheranteil, und zwar in dem Bestreben, das vorhandene Weichmachergefälle auszugleichen. Es wird also gewissermaßen zwischen den benachbarten Wandbereichen ein Weichmacherdruckunterschied geschaffen, der aufgrund der höheren Anteile in der einen Wandung den osmotischen oder Differenzdruckausgleich zur anderen Wandung anstrebt.

Diesem Druckausgleichsbestreben der Weichmacheranteile steht das Wanderungsbestreben der Zuschlagstoffe im Wandbereich mit den geringeren Weichmacherbestandteilen entgegen, wobei diese Zuschlagstoffe aufgrund des völligen Fehlens solcher Bestandteile in der Schlauchwandung mit den höheren Weichmacherbestandteilen das umgekehrte osmotische Ausgleichsbestreben aufweisen.

Dem Wanderungsbestreben dieser Zuschlagstoffe, bei denen es sich beispielsweise um UV-Absorber, z. B. Derivate des Benzophenons, substituierte Benzotriazole, organische Metallkomplexe, insbesondere des Nickels, Zimtsäurederivate und andere handelsübliche transparente Pigmente oder Farbstoffe oder transparente röntgenkontrastgebende Stoffe wie Äthyl-2(3-Amino-2,4,6)-Triiodobenzoyloxy)-Butanoat handelt, steht jedoch der Druck der Weichmachersubstanzen entgegen, so daß dadurch die Wanderung der Moleküle dieser Zuschlagstoffe aus dem Wandungsbereich mit den geringeren Weichmacheranteilen in den Wandungsbereich mit den höheren Weichmacheranteilen verhindert wird.

3

Der Gefälledruck des Weichmachers von Wandbereich zu Wandbereich kann so aufgebaut sein, daß auch theoretisch ein Druckausgleich während der nach dem Arzneimittelgesetz zulässigen Lagerzeit solcher medizinischen Arbeitsmittel nicht möglich ist.

Werden solche medizinischen Arbeitsmittel mit einem Wandungsaufbau verwendet, bei dem beispielsweise die physiologisch einwandfreie Wandung aus weich eingestelltem Polyvinylchlorid, die benachbarte Wandung dagegen aus einem anderen weichen Polymeren ohne Weichmacherbestandteile besteht, ergibt sich für das Penetrationsverhalten des Weichmachers aus der einen Wandung in die weichmacherfreie Polymer-Wandung ein gleiches Verhalten wie oben beschrieben. Hier kann der Weichmachergehalt der Polyvinylchlorid-Wandung entsprechend den besonderen Anforderungen der benachbarten Wandung aus dem weichmacherfreien Polymer eingestellt werden.

Es gilt die Regel, daß, je größer das Weichmachergefälle von der einen Wandung zur anderen Wandung ist, desto länger wirkt das Gefälle und desto länger wird das Eindringen von physiologisch bedenklichen und/oder ungeprüften Bestandteilen der einen Wandung in die physiologisch einwandfrei eingestellte andere Wandung verhindert. Hier gilt jedoch auch, daß die Maximalgrenze der Beladung von Polyvinylchlorid mit Weichmacherbestandteilen bei 60 Gewichtsprozent liegt.

Das Prinzip kann bei Schläuchen verwendet werden, die z. B. im Koextrusionsverfahren zwei- oder mehrschichtig aufgebaut sind. Andere Verfahrenstechniken zur Herstellung von mehrschichtigen Schläuchen sind hier selbstverständlich erfaßt. Gleiches gilt für andere medizinische Arbeitsmittel wie Infusionsbeutel, Formteile für den Schlauchanschluß usw. Zu beachten ist hierbei jedoch immer, daß ein vollkommener Schichtenabschluß zwischen dem Wandungsteil mit Weichmacher oder mit höherem Weichmachergehalt und dem Wandungsteil mit weniger oder gar keinem Weichmacher erfolgt, damit die Sperrwirkung gegen die Durchwanderung physiologisch bedenklicher und/oder ungeprüfter Zuschlagstoffe gegenüber dem Transport- oder Durchflußgut erhalten bleibt. Die Verbindung der verschiedenen Wandteile bei einem mehrschichtigen Wandaufbau eines solchen medizinischen Arbeitsmittels kann beispiels weise durch Schweißen, Kleben, im Wege der Koextrusion usw. erfolgen.

Als Weichmacher für die Wandungsbereiche aus Polyvinylchlorid kann hier z. B. das von der DIN 58 361 zugelassene Di-(2-Äthylhexyl)-Phthalat (DOP) verwendet werden. Es kommen jedoch auch Weichmacher wie Di-oktyl Adipate oder Polymerweichmacher auf Basis Adipinsäure in Frage. Wesentlich hierbei ist eine entsprechende Abstimmung auf das Durchfluß- oder Transportgut, um Auswaschungen weitgehend zu verhindern. Solche Auswaschungen können die Weichmacherbestandteile der einen Wandung verrigern und damit den Druckaufbau zwischen benachbarten Wandbereichen negativ beeinflussen. Dies könnte insbesondere dort der Fall sein, wo erfindungsgemäß ein Schlauch derart aufgebaut ist, daß dessen Innenwandung aus einem physiologisch einwandfreien Polyvinylchlorid weich besteht, welche von einer Außenwand mit physiologisch bedenklichen und/oder ungeprüften Zuschlagstoffen umgeben ist.

Neben dem Weichmachergefälle zwischen benachbarten Wandbereichen ist die Verwendung von Absorbersubstanzen in Wandbereichen eines medizinischen Arbeitsmittels aus Kunststoff wichtig, wobei diese Absorbersubstanzen Teilbereiche des sichtbaren und/oder unsichtbaren Lichtes ausfiltern,ohne die Transparenz des Mehrschichtenaufbaus des Arbeitsmittels negativ zu beeinflussen. Es sollen hier die Wellenlängenbereiche im unsichtbaren und im sichtbaren Licht zwischen 220 und 750 Nanometer abgedeckt werden. Grund hierfür ist die Lichtempfindlichkeit des Durchfluß- oder Transportgutes, welches sich bei fehlendem Schutz entweder in einem photochemischen Abbau oder in einem durch Energie katalysierten, oxidativen Abbau durch Aktivierung in einem bestimmten Wellenlängenbereich auswirken kann. Zusätzlich zu den genannten Nanometer-Bereichen kann durch entsprechende Zuschläge der Röntgenstrahlenbereich von 0,01 bis 50 Nanometer abgedeckt werden. Im Einzelfall kann durch verschiedene Absorberzusätze jeder Wellenlängenbereich des sichtbaren Lichtes erfaßt und dabei dennoch die Forderung nach Beibehaltung der für eine Beobachtung des Durchfluß- bzw. Transportgutes erforderlichen Transparenz erfüllt werden. Das gleiche gilt für die angegebenen Wellenlängenbereiche des unsichtbaren Lichtes sowie die genannten Röntgenstrahlenbereiche.

Die Breite der Erfindung soll nachstehend durch verschiedene Beispiele unterstrichen werden :

Beispiel 1

Es wurde ein Verbundschlauch mit einem Innendurchmesser von 4,5 mm und einer Wandstärke von 1,35 mm hergestellt. Zur Rezeptur des Mantelschlauches wurden folgende Bestandteile verwendet.

| 100 | Teile | S-PVC |
|---|---|---|
| 10 | Teile | Weichmacher |
| 2 | Teile | Kalzium/Zinkstabilisator (Ca/Zn) |
| 5 | Teile | Epoxidiertes Sojaöl |
| 0,06 | Teile | Euvenylblau |
| 0,20 | Teile | Cyasorb UV 24 |

Der Schlauchmantel aus dieser Rezeptur filtert 100 % des Lichts der Wellenlängen 220 bis 376 mm aus. Davon entfallen auf das sichtbare licht die Bereiche 350 bis 376 mm und auf den Ultraviolettbereich 220 bis 350 mm. Aufgrund der zugegebenen Zuschlagstoffe ist der Mantelschlauch physiologisch bedenklich. Seine Schichtdicke betrug 0,7 mm.

Die Rezeptur des Innenschlauches hatte folgenden Aufbau :

| 100 Teile | S-PVC |
| 50 Teile | Weichmacher |
| 2 Teile | Ca/Zn-Stabilisator |
| 5 Teile | Epoxidiertes Sojaöl |

Die Schichtdicke dieses Innenschlauches betrug 0,65 mm, aufgrund seiner Zuschlagstoffe ist er als physiologisch einwandfrei zu bezeichnen.

Mit diesem Schlauch können Durchflußgüter befördert werden, welche gegen die angegebenen Bereiche der Wellenlängen des sichtbaren und des unsichtbaren Lichtes anfällig sind. Diese Bereiche können verändert werden, indem das Euvenylblau des Beispiels durch beliebige andere Farbstoffe ersetzt wird. Hier kommt z. B. folgende Palette in Betracht :

| Irgazingelb | nm-Bereiche 380 bis 510 |
| Irgazinorange | nm-Bereiche 380 bis 540 |
| Irgazinrot | nm-Bereiche 450 bis 570 |
| Irgazinviolett | nm-Bereiche 520 bis 560 |
| Irgazinblau | nm-Bereiche 580 bis 750 |

Es können eine oder mehrere dieser Farbstoffe einzeln oder in Kombination miteinander eingesetzt werden.

Beispiel 2

Hier wurde ein Verbundschlauch mit einem Innendurchmesser von 4,5 mm und einer Wandstärke von 1,35 mm erzeugt. Bei der Rezeptur des Außenschlauches wurden folgende Bestandteile verwendet :

| 100 Teile | S-PVC |
| 50 Teile | Weichmacher |
| 2 Teile | Ca/Zn-Stabilisator |
| 5 Teile | Epoxidierte Sojaöl |

Dieser Schlauch erhielt eine Schichtdicke von 0,4 mm. Er ist aufgrund seiner Bestandteile als physiologisch einwandfrei zu bezeichnen.

Im Koextrusionsverfahren wurde diesem Schlauch ein Deckschlauch aufextrudiert mit folgender Rezeptur :

| 100 Teile | S-PVC |
| 15 Teile | Weichmacher |
| 2 Teile | Ca/Zn-Stabilisator |
| 5 Teile | Epoxidiertes Sojaöl |
| 0,2 Teile | Cyasorb UV 24 |

Dieser Schlauchmantel filtert 100 % der Welllängen zwischen 220 bis 350 mm und ist als physiologisch bedenklich zu bezeichnen aufgrund seiner Zuschlagstoffe.

Der letztgenannte Schlauch erhielt ebenfalls im Koextrusionsverfahren einen Außenschlauch, überzogen mit nachfolgender Rezeptur :

| 100 Teile | S-PVC |
| 50 Teile | Weichmacher |
| 2 Teile | Ca/Zn-Stabilisator |
| 5 Teile | Epoxidiertes Sojaöl |

Die Schichtdicke dieses Außenschlauches betrug 0,4 mm und es bleibt nachzutragen, daß die Schichtdicke des Mittelschlauches 0,55 mm betragen hat.

Im Beispiel 2 ist der Außen- und der Innenschlauch physiologisch unbedenklich eingestellt, während der Mittelschlauch die physiologisch bedenklichen Substanzen enthält.

Das Weichmachergefälle vom Innenschlauch zum Mittel schlauch sowie vom Außenschlauch zum Mittelschlauch verhindert hier jedoch das Durchdringen der gefährlichen Substanzen des Mittelschlauches sowohl nach innen als auch nach außen. Dieser Schlauch kann zum Transport vom Durchflußgut genauso eingesetzt werden wie beispielsweise als Drainageschlauch für die Wunddrainage, wo der Außenschlauch mit der Wunde in Kontakt steht und einwandfrei sein muß.

### Beispiel 3

Hier wurde ein Verbundschlauch mit einem Innendurchmesser von 3,0 mm und einer Wandstärke von 0,55 mm hergestellt.

Die Rezeptur des Außenschlauches setzte sich wie folgt zusammen :

| 100 Teile | S-PVC |
| 18 Teile | Weichmacher |
| 2 Teile | Ca/Zn-Stabilisator |
| 5 Teile | Epoxidiertes Sojaöl |
| 0,3 Teile | Cyasorb UV 24 |

Dieser Schlauch hatte eine Schichtdicke von 0,2 mm und filtert 100 % der Wellenlängen von 220 bis 350 mm aus. Der Schlauchaufbau·ist aufgrund seiner Bestandteile als physiologisch bedenklich einzustufen.

Diesem Außenschlauch wurde ein Innenschlauch zugespritzt mit einer Schichtdicke von 0,35 mm und einer Rezeptur wie folgt :

| 100 Teile | · S-PVC |
| 50 Teile | Weichmacher |
| 2 Teile | Ca/Za-Stabilisator |
| 5 Teile | Epoxidiertes Sojaöl |

Der Innenschlauch ist auch hier physiologisch unbedenklich.

### Beispiel 4

Es wurde ein Verbundschlauch gefertigt mit Innendurchmesser 3,0 mm und einer Wandstärke von 0,55 mm.

Die Rezeptur des Innenschlauches setzte sich wie folgt zusammen :

| 100 Teile | S-PVC |
| 14 Teile | 2-Ethoxythyl-2,3,4-tetra-iodobenzoat |
| 8 Teile | Epoxidiertes Sojaöl |
| 2 Teile | Ca/Zn-Stabilisator |

Die Schichtdicke des Innenschlauches betrug 0,2 mm und er ist aufgrund seiner Zuschlagstoffe als physiologisch nicht einwandfrei einzuordnen. Der als Röntgenkontrastmittel eingebrachte Zuschlagstoff 2-Ethoxyethyl-2,3,4,6-tetra-iodobenzoat absorbiert die Röntgenstrahlung und kann somit im Körper eines Patienten geortet werden.

Diesem Innenschlauch wurde ein Außenschlauch zugespritzt mit folgender Rezeptur :

| 100 Teile | S-PVC |
| 50 Teile | Weichmacher |
| 2 Teile | Ca/Zn-Stabilisator |
| 8 Teile | Epoxidiertes Sojaöl |

Dieser Außenschlauch erhielt eine Schichtdicke von 0,35 mm und war physiologisch einwandfrei. Auch hier macht sich das erfindungsgemäße Weichmachergefälle insofern deutlich, als die höheren Bestandteile des Außenschlauches das Durchdringen des Röntgenkontrastmittels an die Oberfläche verhindern. Dieser Schlauch kann als Wunddrainage eingesetzt werden, da seine Außenhaut physiologisch einwandfrei ist.

### Beispiel 5

Hier wurde ein Verbundschlauch mit einem Innendurchmesser von 4 mm und einer Wandstärke von 1 mm hergestellt. Der Schlauch besteht aus vier Schichten mit folgendem Aufbau :

Ein Außenschlauch mit nachfolgender Rezeptur :

| 100 Teile | S-PVC |
| 50 Teile | Weichmacher |
| 2 Teile | Ca/Zn-Stabilisator |
| 8 Teile | Epoxidiertes Sojaöl |

Dieser Außenschlauch erhielt eine Schichtdicke von 0,2 mm und war aufgrund seiner Einstellung als physiologisch einwandfrei zu bezeichnen.

Mit diesem Schlauch verbunden wurde ein Absorberschlauch folgender Rezeptur :

| | | |
|---|---|---|
| 100 | Teile | S-PVC |
| 18 | Teile | Weichmacher |
| 2 | Teile | Ca/Zn-Stabilisator |
| 8 | Teile | Epoxidiertes Sojaöl |
| 0,3 Teile | | Cyasorb UV 24 |

Dieser Absorberschlauch erhielt eine Schichtdicke von 0,2 mm, und er absorbiert aufgrund seiner Zuschlagstoffe im Ultraviolettbereich die Wellenlängen von 220 bis 350 nm, wodurch das in der nächsten Schlauchschicht eingebettete Röntgenkontrastmittel vor den schädlichen Ultraviolett-Strahlen geschützt wird. Der Schlauch selbst ist aufgrund seines Aufbaues als physiologisch bedenklich zu bezeichnen.

Darunter wurde im Schlauchaufbau ein Kontrastschlauch gespritzt mit folgender Rezeptur :

| | |
|---|---|
| 100 Teile | S-PVC |
| 18 Teile | Weichmacher |
| 15 Teile | 1,10-Diioddecan |
| 2 Teile | Ca/Zn-Stabilisator |
| 8 Teile | Epoxidiertes Sojaöl |

Dieser Kontrastschlauch erhielt eine Schichtdicke von 0,2 mm und absorbiert Röntgenstrahlung, wobei er farblos glasklar bleibt, weil der vorgebaute Ultraviolett-Filter die Jodabspaltung verhindert. Der Schlauchaufbau ist aufgrund seiner Zuschlagstoffe als physiologisch bedenklich zu bezeichnen.

Der Verbundschlauch erhielt schließlich einen Innenschlauch mit folgender Rezeptur :

| | |
|---|---|
| 100 Teile | S-PVC |
| 50 Teile | Weichmacher |
| 2 Teile | Ca/Zn-Stabilisator |
| 8 Teile | Epoxidiertes Sojaöl |

Die Schichtdicke des Innenschlauches betrug 0,4 mm und der Innenschlauch ist als physiologisch unbedenklich zu bezeichnen.

Bei diesem Beispiel wird verdeutlicht, daß hier mehrschichtige Aufbauten im Bereich der Erfindung liegen, wobei mehrere Schlauchschichten mit physiologisch bedenklichen Zuschlagstoffen von Schlauchschichten in physiologisch einwandfreier Einstellung eingeschlossen sein können. Dabei können die inneren Schlauchschichten mit physiologisch bedenklichen Zuschlagstoffen sich gegenseitig oder einseitig schützen, wie es beispielsweise der Absorberschlauch des Beispiels gegenüber dem Kontrastschlauch tut.

## Beispiel 6

Es wurde ein Verbundschlauch mit einem Innendurchmesser von 3 mm und einer Wandstärke von 1 mm hergestellt.

Die Rezeptur des Außenschlauches setzte sich wie folgt zusammen :

| | | |
|---|---|---|
| 100 | Teile | Polyurethan |
| 0,6 Teile | | Euvenylblau |
| 3 | Teile | Cyasorb UV 24 |

Dieser Schlauch hatte eine Schichtdicke von 0,4 mm und absorbiert das Licht der Wellenlängen 220 bis 370 nm zu 100 %.

Der zugeordnete Innenschlauch hatte eine Schichtdicke von 0,6 mm und eine Rezeptur wie folgt :

| | |
|---|---|
| 100 Teile | S-PVC |
| 50 Teile | Weichmacher |
| 2 Teile | Ca/Zn-Stabilisator |
| 5 Teile | Epoxidiertes Sojaöl |

Der Innenschlauch ist im Gegensatz zum Außenschlauch physiologisch einwandfrei.

## Beispiel 7

Es wurde ein Verbundschlauch mit einem Innendurchmesser von 2,5 mm und einer Wanddicke von 0,75 mm hergestellt.

Die Rezeptur des Außenschlauches entsprach der des Beispiels 2. Die Schichtdicke betrug 0,3 mm und der Schlauch war physiologisch einwandfrei.

Dieser Außenschlauch erhielt einen Innenschlauch zugeordnet mit folgender Rezeptur:

| | |
|---|---|
| 100 Teile | Äthylen-Vinyl-Acetat (EVA) mit 12 % Vinylacetat |
| 140 Teile | 1,10 Diioddecan |
| 3 Teile | Cyasorb UV 24 |

Die Schichtdicke betrug 0,45 %. Der Innenschlauch absorbierte das UV-Licht und war darüber hinaus röntgenkontratsgebend.

## Beispiel 8

Es wurde ein Verbundschlauch hergestellt mit einem Innendurchmesser von 4 mm und einer Wandstärke von 1 mm.

Der Außenschlauch besaß folgende Rezeptur:

| | |
|---|---|
| 100 Teile | Polydimethylsiloxan mit 3 % Dichlorbenzoylperoxid |
| 3 Teile | Cyasorb UV 24 |

Die Schichtdicke war 0,2 mm und wurde aufgetaucht. Der Schlauch absorbiert das UV-Licht von 220 bis 350 nm.

Der Innenschlauch hatte die Rezeptur des Innenschlauches wie Beispiel 1, seine Schichtdicke betrug 0,8 mm.

Die Beispiele 6-8 zeigen, daß die Erfindung auch in solchen Fällen wirksam eingesetzt werden kann, in denen nur eine Schicht aus weichgemachtem Polyvinylchlorid besteht. Diese Schlauchschicht ist die physiologisch einwandfreie, die mit ihrem Weichmachergefälle gegen die Bestandteile des physiologisch bedenklichen Deckschlauches drückt und das Übertreten dieser Bestandteile aus dem weichmacherfreien Polymerschlauch durch den weichmacherhaltigen Schlauch aus Polyvinylchlorid in das Durchflußgut verhindert.

## Beispiel 9

Hier wurde ein physiologisch einwandfreier Innenschlauch mit einem Mantelschlauch versehen, welcher neben den anderen Zuschlägen ein chemisches Vernetzungsmittel für die nachfolgende chemische Vernetzung enthielt.

Bei der Rezeptur des Innenschlauches wurden folgende Bestandteile verwendet:

| | |
|---|---|
| 100 Teile | S-PVC |
| 50 Teile | Weichmacher |
| 2 Teile | Ca/Zn-Stabilisator |
| 5 Teile | Epoxidiertes Sojaöl |

Der Mantelschlauch dieses Beispiels wies folgende Rezeptur auf:

| | |
|---|---|
| 50 Teile | S-PVC |
| 50 Teile | Äthylenvinylacetat Copolymer mit 60-70 % Vinylacetat |
| 5 Teile | Ca/Zn-Stabilisator |
| 1,5 Teile | Stearinsäure |
| 10 Teile | Weichmacher |
| 5 Teile | Epoxidiertes Sojaöl |
| 2 Teile | 2,2-(Tert.-Butylperoxi) Butan als chemischen Vernetzer |

Der Außenschlauch wurde im Anschluß an die Herstellung zur Erzielung der geschilderten optimalen Eigenschaften chemisch vernetzt.

## Beispiel 10

Bei diesem Beispiel erhielt der Innenschlauch folgende Rezeptur:

| | |
|---|---|
| 100 Teile | S-PVC |

| | |
|---|---|
| 16 Teile | Weichmacher |
| 2 Teile | Ca/Zn-Stabilisator |
| 5 Teile | Epoxidiertes Sojaöl |

Die Rezeptur des Außenschlauches enthielt folgende Bestandteile :

| | | |
|---|---|---|
| 100 | Teile | Äthylenvinylacetat Copolymer |
| 42 | Teile | S-PVC |
| 15 | Teile | Weichmacher |
| 3 | Teile | Epoxidiertes Sojaöl |
| 2 | Teile | Ca/Zn-Stabilisator |
| 3 | Teile | 2,2-(Tert.-Butylperoxi)-Butan |
| 1,5 | Teile | Stearinsäure |
| 2 | Teile | Triallylcyanurat als Vernetzungshilfsmittel |

Auch hier wurde der Außenschlauch nach der Herstellung vernetzt. Die beiden in den Beispielen 9 und 10 genannten chemischen Vernetzungsmittel sind keine erschöpfende Aufzählung. Hier kann jeder gängige chemische Vernetzer eingesetzt werden.

Beispiel 11

Für eine nachfolgende Strahlenvernetzung wurde ein Schlauch aufbau wie folgt ausgewählt : Der Innenschlauch erhielt folgende Rezeptur :

| | |
|---|---|
| 100 Teile | S-PVC |
| 16 Teile | Weichmacher |
| 4 Teile | Ca/Zn-Stabilisator |
| 6 Teile | Epoxidiertes Sojaöl |

Der Außenschlauch war wie folgt zusammengesetzt :

| | | |
|---|---|---|
| 50 | Teile | S-PVC |
| 50 | Teile | Äthylenvinylacetat Copolymer |
| 5 | Teile | Ca/Zn-Stabilisator |
| 1,5 | Teile | Stearinsäure |
| 10 | Teile | Weichmacher |
| 3 | Teile | Triallylcyanurat |

Die Strahlenvernetzung erfolgte hier mit einem Bestrahlungsgrad von 5 Mrd.

**Patentansprüche**

1. Technisches Zubehör für medizinische Arbeitsgeräte wie Schläuche, Behälter, Formteile mit mehrschichtigen Wandungen aus glasklarem, weichem Kunststoff, wobei wenigstens eine der Wandungen physiologisch einwandfrei und die anderen mit physiologisch bedenklichen und/oder ungeprüften Zuschlagstoffen, z. B. radioopakem Material, Farbbestandteilen, versehen sind, dadurch gekennzeichnet, daß die physiologisch einwandfreie Wand jeweils einen höheren Weichmachergehalt besitzt als die dieser Wand benachbarte Wand, in der die physiologisch bedenklichen und/oder ungeprüften Zuschlagstoffe eingebracht sind, derart, daß das verstärkte Migrationsbestreben des Weichmachers aus der physiologisch einwandfreien Wand die Migration der physiologisch bedenklichen und/oder ungeprüften Zuschlagstoffe in die physiologisch einwandfreie Wand verhindert.

2. Technisches Zubehör für medizinische Arbeitsgeräte nach Anspruch 1, dadurch gekennzeichnet, daß bei Verwendung von weich eingestelltem Polyvinylchlorid die Weichmacheranteile zwischen der physiologisch einwandfreien und der physiologisch bedenklichen Wand in einem Verhältnis von 1,1 : 1 bis 9 : 1 stehen.

3. Technisches Zubehör für medizinische Arbeitsgeräte nach Anspruch 1, dadurch gekennzeichnet, daß bei einer Kombination von weich eingestelltem Polyvinylchlorid mit anderen weichen Polymeren der Weichmacheranteil des Polyvinylchlorids wenigstens 7 Gewichtsprozent ist.

4. Technisches Zubehör für medizinische Arbeitsgeräte nach Anspruch 1, dadurch gekennzeichnet, daß die Zuschlagstoffe strahlenabsorbierende Substanzen sind.

5. Technisches Zubehör für medizinische Arbeitsgeräte nach Anspruch 1 oder 4, dadurch gekennzeichnet, daß die Zuschlagstoffe Strahlen im Wellenlängenbereich des sichtbaren Lichtes absorbieren.

6. Technisches Zubehör für medizinische Arbeitsgeräte nach Anspruch 1, 4 oder 5, dadurch gekennzeichnet, daß die Zuschlagstoffe Strahlen im Wellenlängenbereich des unsichtbaren Lichtes

9

absorbieren.

7. Technisches Zubehör für medizinische Arbeitsgeräte nach Anspruch 1, 4, 5 oder 6, dadurch gekennzeichnet, daß die Zuschlagstoffe Strahlen im Wellenlängenbereich der Röntgenstrahlung absorbieren.

## Claims

1. Technical accessories for medical equipment, as for instance, hoses, tanks, mouldings with multilayer walls made of highly transparent, soft plastic, with at least one of the walls physiologically unobjectionable, and the others containing physiologically questionable and/or untested additives, e. g., radiopaque material, colorants, are characterized by the fact that the physiologically unobjectionable wall has always a higher plasticizer content than its neighbouring wall, which contains the physiologically questionable and/or untested additives, and this in such a way that the increased migration tendency out of the physiologically unobjectionable wall of the plasticizer impedes the migration of the physiologically questionable and/or untested additives into the physiologically unobjectionable wall.

2. Technical accessories for medical equipment according to Claim1, characterized by the fact that, if soft polyvinyl chloride is used, the contents of plastizicers of the physiologically unobjectionable and the physiologically questionable wall are in a ratio of 1.1 to 1 till 9 to 1.

3. Technical accessories for medical equipment according to Claim 1, characterized by the fact that in the case of a combination of soft polyvinyl chloride with other solft polymers, the plasticizer content of the polyvinyl chloride is at least seven (7) percent by weight.

4. Technical accessories for medical equipment according to Claim 1, characterized by the fact that the additives are radiation absorbing substances.

5. Technical accessories for medical equipment according to Claim 1, or 4, characterized by the fact that the additives absorb rays within the range of wave lengths of visible light.

6. Technical accessories for medical equipment according to Claim 1, 4 or 5, characterized by the fact that the additives absorb rays within the range of wave lengths of invisible light.

7. Technical accessories for medical equipment according to Claim 1, 4, 5 or 6, characterized by the fact that the additives absorb rays within the range of wave lengths of X-rays.

## Revendications

1. Accessoires techniques pour matériel médical tels que tuyaux flexibles, récipients, pièces moulées possédant des parois multicouches en matière plastique souple et transparente, l'une des parois au moins étant physiologiquement neutre et les autres munies de matières de charge physiologiquement douteuses et/ou non testées, comme par exemple matière opaque aux rayons X, constituants colorants, caractérisés par le fait que la paroi physiologiquement neutre possède dans chaque cas une teneur en plastifiant plus élevée que la paroi voisine dans laquelle sont incorporées des matières de charge physiologiquement douteuses et/ou non testées, de telle façon que la tendance renforcée du plastifiant à la migration hors de la paroi physiologiquement neutre empêche la migration des matières de charge physiologiquement douteuses et/ou non testées vers la paroi physiologiquement neutre.

2. Accessoires techniques pour matériel médical selon la spécification 1, caractérisés par le fait que, dans le cas de l'utilisation de polychlorure de vinyle plastifié, les pourcentages de plastifiant de la paroi physiologiquement neutre et de la paroi physiologiquement douteuse sont dans un rapport allant de 1,1 : 1 à 9 : 1.

3. Accessoires techniques pour matériel médical selon la spécification 1, caractérisés par le fait que, dans le cas d'une combinaison de polychlorure de vinyle plastifié et d'autres polymères souples, le pourcentage de plastifiant du polychlorure de vinyle est au moins de 7 % en poids.

4. Accessoires techniques pour matériel médical selon la spécification 1, caractérisés par le fait que les matières de charge sont des substances absorbant des rayonnements.

5. Accessoires techniques pour matériel médical selon les spécifications 1 ou 4, caractérisés par le fait que les matières de charge absorbent des rayonnements dans la gamme de longueurs d'ondes de la lumière visible.

6. Accessoires techniques pour matériel médical selon les spécifications 1, 4 ou 5, caractérisés par le fait que les matières de charge absorbent des rayonnements dans la gamme de longueurs d'ondes de la lumière invisible.

7. Accessoires techniques pour matériel médical selon les spécifications 1, 4, 5 ou 6, caractérisés par le fait que les matières de charge absorbent des rayonnements dans la gamme de longueurs d'ondes du rayonnement X.